# EUROPEAN PATENT SPECIFICATION

(11) **EP 3 320 885 B1**
(45) Date of publication and mention of the grant of the patent: **11.03.2020**
(21) Application number: 16841263.3
(22) Date of filing: 30.06.2016
(51) Int. Cl.: A61F 13/15, B29C 65/00

(54) **METHOD FOR PRODUCING COMPOSITE SHEET FOR ABSORBENT ARTICLE, APPARATUS FOR PRODUCING COMPOSITE SHEET FOR ABSORBENT ARTICLE, AND COMPOSITE SHEET FOR ABSORBENT ARTICLE**
VERFAHREN ZUR HERSTELLUNG EINER VERBUNDFOLIE FÜR EINEN SAUGFÄHIGEN ARTIKEL, VORRICHTUNG ZUR HERSTELLUNG EINER VERBUNDFOLIE FÜR EINEN SAUGFÄHIGEN ARTIKEL UND VERBUNDFOLIE FÜR EINEN SAUGFÄHIGEN ARTIKEL
PROCÉDÉ ET APPAREIL DE PRODUCTION D'UNE FEUILLE COMPOSITE POUR ARTICLE ABSORBANT, ET FEUILLE COMPOSITE POUR ARTICLE ABSORBANT

(30) Priority: 02.09.2015 JP 2015173101
(43) Date of publication of application: 16.05.2018
(73) Proprietor: Unicharm Corporation, Ehime 799-0111 (JP)
(72) Inventor: OHTSUBO, Toshifumi, Kanonji-shi Kagawa 769-1602 (JP)
(74) Representative: Dolleymores
(86) International application number: PCT/JP2016/069458
(87) International publication number: WO 2017/038231

(56) References cited:
- WO-A1-2005/025789
- WO-A1-2014/141913
- GB-A- 2 257 652
- JP-A- H0 515 551
- JP-A- 2000 014 701
- JP-A- 2001 080 016
- JP-A- 2008 132 023
- JP-A- 2008 148 834
- JP-A- 2011 229 907

## Description

### Technical Field

The present invention relates to methods for manufacturing a composite sheet of an absorbent article, apparatus for manufacturing a composite sheet of an absorbent article, and composite sheets of an absorbent article.

### Background

A disposable diaper is known as an absorbent article that absorbs excrement such as urine. This disposable diaper is formed from an exterior member (exterior body) having an interior sheet, an exterior sheet, and an elastic string (elastic member) and an absorbent main body, and as shown in Document 1, the disposable diaper is known to be processed by forming through holes in the exterior member to improve permeability and the like.

### Citation List

### Patent Literature

PTL 1: Japanese Patent Application Publication No. 2015-107223

JP 2008 132023 A and JP 2008 148834 A relate to pants-type absorbent articles having waist gathers.

WO 2014/141913 A1 discloses a stretchable sheet manufacturing method including a line stoppage step.

JP 2001 080016 A relates to a method for producing a composite sheet.

GB 2257652 A provides a method and an apparatus for joining a side part around the waist of a wearing article, where elastic members between around the waist and the leg are arranged between protrusions of an anvil.

WO 2005/025789 A1 discloses a composite extensible sheet member having pleats.

### Summary

### Technical Problem

It is necessary, however, to form the through hole between an elastic string and an elastic string, but when a position to perform processing such as to form a through hole is displaced, the elastic string could be erroneously cut, and there is a possibility that tension of the elastic string is decreased. Further, there is a possibility that the elastic string might be cut not only in forming the through hole, but also similarly in crimping, welding, fusion bonding and the like.

The present invention has been made in view of the above issues, and an object is to decrease the possibility of cutting an elastic string erroneously, during processing of a composite sheet that forms an exterior member.

### Solution to Problem

The present invention provides the method for manufacturing a composite sheet of an absorbent article of independent claim 1 and the apparatus for manufacturing a composite sheet of an absorbent article of independent claim 13. The dependent claims specify preferred but optional features.

In a method for manufacturing a composite sheet of an absorbent article to manufacture a composite sheet including a first continuous sheet, a second continuous sheet, and an elastic string to be arranged between the first continuous sheet and the second continuous sheet, the method for manufacturing a composite sheet of an absorbent article includes: transporting the first continuous sheet, the second continuous sheet, and the elastic string, in a state where the elastic string arranged between the first continuous sheet and the second continuous sheet is at least partially interposed between first protruding parts and second protruding parts, by causing a rotatable first rotation body to rotate, the first rotation body including the first protruding parts and the second protruding parts protruding radially outwardly; and performing processing to the first continuous sheet and the second continuous sheet by sandwiching the first continuous sheet and the second continuous sheet between a rotatable second rotation body and the first protruding parts and the second protruding parts, during transporting with the first rotation body.

Other features of this invention will become clear from the description of this specification and attached drawings.

### Advantageous Effects of Invention

According to the present invention, transporting the first continuous sheet, the second continuous sheet, and the elastic string, in a state where the elastic string is at least partially interposed between first protruding parts and second protruding parts of a first rotation body, and by sandwiching the first continuous sheet and the second continuous sheet between a second rotation body and the first protruding parts and the second protruding parts, movement of the elastic string during transporting can be restricted, and the possibility of the elastic string being cut in processing of sandwiching the first continuous sheet and the second continuous sheet can be decreased.

### Brief Description of Drawings

[Fig. 1] Fig. 1 is a schematic front view of a diaper 1 of a first embodiment.
[Fig. 2] Fig. 2A is a plan view of a diaper 1 in an unfolded state. Fig. 2B is a cross-sectional view that is a cross-sectional view taken along a line A-A in Fig. 2A.
[Fig. 3] Fig. 3 is a schematic view describing an apparatus 2 for manufacturing a composite sheet 20 in a first embodiment.
[Fig. 4] Fig. 4A is a schematic central sectional view describing a processing apparatus 30 during processing. Fig. 4B is a schematic central sectional view describing a processing apparatus 30 that has stopped processing.
[Fig. 5] Fig. 5A is a view showing a part of an outer peripheral surface of a process roller 25 of a first embodiment. Fig. 5B is a schematic perspective view of one first protruding part 251 in Fig. 5A. Fig. 5C is a schematic perspective view of one modification of a first protruding part 251.
[Fig. 6] Fig. 6 is a schematic view of one modification of a first protruding part 251 as viewed from an axial direction of a rotation shaft 250.
[Fig. 7] Fig. 7A is a view taken along arrows B-B in Fig. 3. Fig. 7B is a view taken along arrows B-B in Fig. 3 in a modified example.
[Fig. 8] Fig. 8 is a schematic diagram describing an apparatus 2 that has stopped processing.
[Fig. 9] Fig. 9 is a view showing a part of an outer peripheral surface of a process roller 25 in another embodiment.
[Fig. 10] Fig. 10A is a view describing an apparatus 2' for manufacturing a composite sheet 20 in the other embodiment. Fig. 10B is a view taken along arrows D-D in Fig. 10A.

### Description of Embodiments

At least below matters will become clear from descriptions in this specification and attached drawings.

A method for manufacturing a composite sheet of an absorbent article to manufacture a composite sheet including a first continuous sheet, a second continuous sheet, and an elastic string to be arranged between the first continuous sheet and the second continuous sheet, the method for manufacturing a composite sheet of an absorbent article including: transporting the first continuous sheet, the second continuous sheet, and the elastic string, in a state where the elastic string arranged between the first continuous sheet and the second continuous sheet is at least partially interposed between first protruding parts and second protruding parts, by causing a rotatable first rotation body to rotate, the first rotation body including the first protruding parts and the second protruding parts protruding radially outwardly; and performing processing to the first continuous sheet and the second continuous sheet by sandwiching the first continuous sheet and the second continuous sheet between a rotatable second rotation body and the first protruding parts and the second protruding parts, during transporting with the first rotation body.

According to such a method for manufacturing a composite sheet of an absorbent article, transporting the first continuous sheet, the second continuous sheet and the elastic string, in a state where the elastic string is at least partially interposed between first protruding parts and second protruding parts, and by sandwiching the first continuous sheet and the second continuous sheet between a second rotation body and first protruding parts or second protruding part, during transporting, movement of the elastic string can be restricted while transporting, and the possibility of the elastic string being cut with processing by sandwiching the first continuous sheet and the second continuous sheet can be decreased.

A method for manufacturing a composite sheet of an absorbent article, wherein while rotation of the first rotation body is stopped, a continuous sheet positioned to the first rotation body side of the first continuous sheet and the second continuous sheet is placed apart from the first rotation body by merely a predetermined distance.

According to such a method for manufacturing a composite sheet of an absorbent article, while rotation of the first rotation body is stopped, a sheet positioned to the first rotation body side is placed apart from the first rotation body, thus a malfunction due to the continuous sheet positioned to the first rotation body side contacting the first rotation body, while transporting is stopped, can be decreased.

A method for manufacturing a composite sheet of an absorbent article, wherein preferably during the processing, the first protruding parts and the second protruding parts heat the first continuous sheet and the second continuous sheet.

According to such a method for manufacturing a composite sheet of an absorbent article, in the case of processing the first and the second continuous sheets by heating, while transporting is stopped, the continuous sheet positioned to the first rotation body side is placed apart from the first rotation body, thus excessive heating of the first and the second continuous sheets can be prevented.

A method for manufacturing a composite sheet of an absorbent article, wherein preferably while rotation of the first rotation body is stopped, a distance between the first rotation body and the second rotation body is wider than a distance between the first rotation body and the second rotation body during the processing.

According to such a method for manufacturing a composite sheet of an absorbent article, while rotation of the first rotation body is stopped, a distance between the first rotation body and the second rotation body is made wider than during the processing, thus a malfunction due to the continuous sheet positioned to the first rotation body side contacting the first rotation body can be further decreased.

A method for manufacturing a composite sheet of an absorbent article, wherein preferably while rotation of the first rotation body is stopped, a transfer path located immediately before contacting the first rotation body, of a transfer path of a continuous sheet positioned to the first rotation body side of the first continuous sheet and the second continuous sheet, is in a state changed to a direction apart from the first rotation body.

According to such a method for manufacturing a composite sheet of an absorbent article, while the first rotation body is stopped, a transfer path located immediately before contacting the first rotation body, of a transfer path of a continuous sheet positioned to the first rotation body side, is in a state changed to a direction apart from the first rotation body. Thus, the continuous sheet and the elastic string can be further placed apart from the first rotation body, and a malfunction due to the continuous sheet positioned to the first rotation body side contacting the first rotation body can be further decreased.

A method for manufacturing a composite sheet of an absorbent article, wherein preferably a transport speed to transport the first continuous sheet, the second continuous sheet, and the elastic string is faster after the processing than before the processing.

According to such a method for manufacturing a composite sheet of an absorbent article, a transport speed is made faster after the processing than before the processing. Thus, before the processing, tension on the first continuous sheet, the second continuous sheet, and the elastic string can be relatively eased, and the elastic string can be easily placed with the first protruding parts and the second protruding parts, whereas after the processing, the first continuous sheet, the second continuous sheet, and the elastic string, can be relatively tensioned, and positional displacement caused by transporting can be decreased and to transport can be carried out efficiently.

A method for manufacturing a composite sheet of an absorbent article, wherein preferably the elastic string is arranged between the first continuous sheet and the second continuous sheet in a state attached with an adhesive.

According to such a method for manufacturing a composite sheet of an absorbent article, the elastic string is arranged between the first continuous sheet and the second continuous sheet in a state attached with an adhesive, thus the possibility of the adhesive leaking out from the edges of the first continuous sheet and the second continuous sheet can be decreased than in the case where an adhesive is applied between the first continuous sheet and the second continuous sheet.

A method for manufacturing a composite sheet of an absorbent article, wherein preferably an adhesive is in a state attached between the elastic string and a continuous sheet positioned to the first rotation body side of the first continuous sheet and the second continuous sheet, the first rotation body includes recessed parts between the first protruding parts and the second protruding parts, and a continuous sheet positioned to the first rotation body side, of the first continuous sheet and the second continuous sheet, is located apart from a most recessed portion of the recessed parts by a predetermined distance.

According to such a method for manufacturing a composite sheet of an absorbent article, a continuous sheet positioned to the first rotation body side is located apart from a most recessed portion of the recessed parts of the first rotation body, thus even when an adhesive is applied to the elastic string or the continuous sheet, the possibility of an adhesive attaching to the first rotation body can be decreased.

A method for manufacturing a composite sheet of an absorbent article, wherein preferably at least one protruding part of the first protruding part and the second protruding part includes a first inclined surface and a second inclined surface in a tip end portion in a protruding direction, in a direction of rotation of the first rotation body, the first inclined surface is provided to a downstream side than the second inclined surface, and when a plane including the first inclined surface is a first plane, and a plane including the second inclined surface is a second plane, a position in which the first plane and the second plane intersect, in the direction of rotation, is positioned to an upstream side than a central position of the at least one protruding part.

According to such a method for manufacturing a composite sheet of an absorbent article, at least one protruding part of the first protruding part and the second protruding part includes a first inclined surface and a second inclined surface in a tip end portion in a protruding direction, in a direction of rotation of the first rotation body, the first inclined surface is provided to a downstream side in a direction of movement than the second inclined surface, and when a plane including the first inclined surface is a first plane, and a plane including the second inclined surface is a second plane, a position in which the first plane and the second plane intersect is positioned to an upstream side in a direction of movement than a central position of the at least one protruding part. Thus, the possibility of the first protruding part and the second protruding part being damaged during processing can be decreased, and damage to the continuous sheet due to processing can be decreased.

A method for manufacturing a composite sheet of an absorbent article, wherein preferably before the processing, a continuous sheet positioned to at least the first rotation body side of the first continuous sheet and the second continuous sheet is heated.

According to such a method for manufacturing a composite sheet of an absorbent article, before the processing, by heating a continuous sheet, the continuous sheet becomes soft, the elastic string can be easily placed in the first rotation body, and positional displacement of the elastic string can be decreased.

A method for manufacturing a composite sheet of an absorbent article, wherein preferably the first rotation body includes a first protruding part group formed from a plurality of protruding parts along a circumferential direction and a second protruding part group formed from a plurality of protruding parts along a circumferential direction, the first protruding part group includes the first protruding parts, the second protruding part group includes the second protruding parts, and each protruding part of the first protruding part group and each protruding part of the second protruding part group are arranged in positions different from each other in an axial direction and the circumferential direction.

According to such a method for manufacturing a composite sheet of an absorbent article, the first rotation body includes a first protruding part group formed from a plurality of protruding parts along a circumferential direction and a second protruding part group formed from a plurality of protruding parts along a circumferential direction, and each protruding part of the first protruding part group and each protruding part of the second protruding part group are arranged in positions different from each other in an axial direction and the circumferential direction. Thus, a part to which processing is performed to at once can be made small, and the possibility that the force during processing is dispersed and processing becomes insufficient can be decreased.

A method for manufacturing a composite sheet of an absorbent article, wherein preferably in a certain position in a direction of rotation of the first rotation body, a plurality of the elastic strings are arranged between the first continuous sheet and the second continuous sheet, the first continuous sheet, the second continuous sheet, and the elastic strings are transported to another position in the direction of rotation using the first rotation body, and processing is performed by sandwiching the first continuous sheet and the second continuous sheet in the other position.

According to such a method for manufacturing a composite sheet of an absorbent article, in the state where the elastic strings are placed between the first protruding parts and the second protruding parts protruding radially outwardly on the first rotation body, the first continuous sheet, the second continuous sheet, and the elastic strings are transported, thus displacement of the elastic strings caused by transporting can be decreased.

An apparatus for manufacturing a composite sheet of an absorbent article to manufacture a composite sheet including a first continuous sheet, a second continuous sheet, and an elastic string arranged between the first continuous sheet and the second continuous sheet, the apparatus for manufacturing including: a transporting part that transports the first continuous sheet, the second continuous sheet, and the elastic string, in a state where the elastic string arranged between the first continuous sheet and the second continuous sheet is at least partially interposed between first protruding parts and second protruding parts, by causing a rotatable first rotation body to rotate, the first rotation body including the first protruding parts and the second protruding parts protruding radially outwardly; and a processing part that processes the first continuous sheet and the second continuous sheet by sandwiching the first continuous sheet and the second continuous sheet between a rotatable second rotation body and the first protruding parts and the second protruding parts, during transporting with the first rotation body.

According to such an apparatus for manufacturing a composite sheet of an absorbent article, the apparatus transports the first continuous sheet, the second continuous sheet, and the elastic string, in a state where the elastic string is at least partially interposed between first protruding parts and second protruding parts of a first rotation body; and sandwiches the first continuous sheet and the second continuous sheet between a second rotation body and the first protruding parts and the second protruding parts, during transporting, thus being able to restrict movement of the elastic strings during transporting, and decrease the possibility of the elastic strings being cut in processing by sandwiching the first continuous sheet and the second continuous sheet.

A composite sheet of an absorbent article including a first continuous sheet, a second continuous sheet, and an elastic string arranged between the first continuous sheet and the second continuous sheet, wherein in the case where the composite sheet is extended in a continuous direction until the second continuous sheet has no slack, the first continuous sheet has slack.

According to such a composite sheet of an absorbent article, in the case where the composite sheet is extended in a continuous direction until the second continuous sheet has no slack, the first continuous sheet has slack. Thus, more creases are formed in the first continuous sheet than in the second continuous sheet, and the first continuous sheet can be made softer.

### ===First embodiment===

### <Basic configuration of Diaper 1>

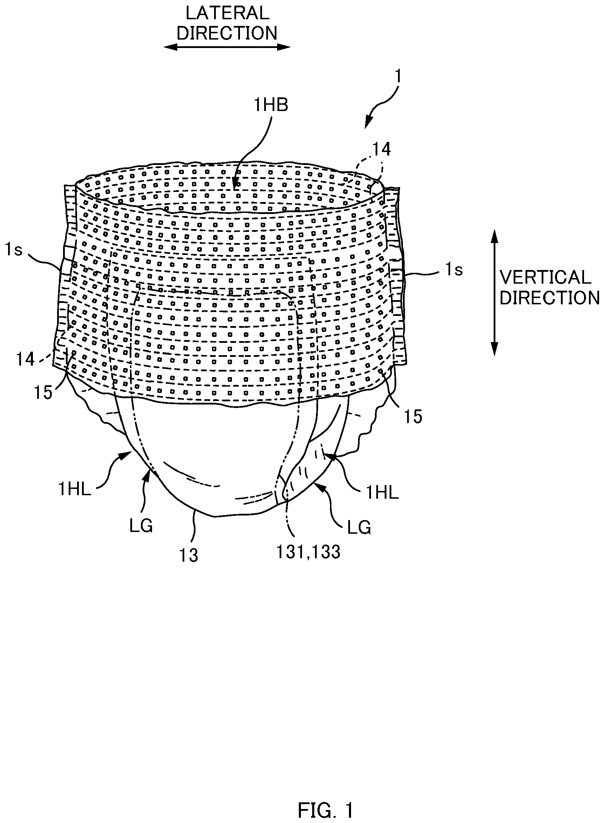

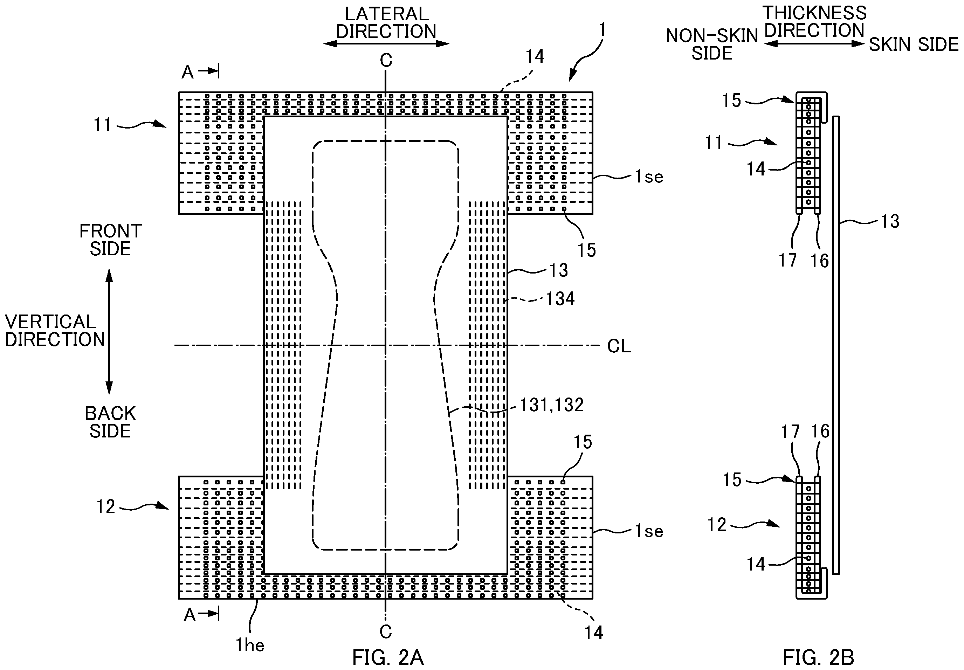

A basic configuration of a pull-on disposable diaper 1 (hereafter, also referred to as "diaper 1") is described as an example of an absorbent article to be manufactured with a method for manufacturing according to the present embodiment. Fig. 1 is a schematic front view of the diaper 1 in a first embodiment. Fig. 2A is a plan view of the diaper 1 in an unfolded state. Fig. 2B is a cross-sectional view that is a cross-sectional view taken along a line A-A in Fig. 2A.

As shown in Fig. 1, the diaper 1 includes a front side member 11 that covers a front side of a wearer, a back side member 12 that covers a back side of a wearer, and an absorbent main body 13 that is arranged in a crotch of a wearer and that absorbs excrement. The diaper 1 is formed with a waist opening 1HB and a pair of leg openings 1HL, 1HL. The diaper 1 is formed by folding in two the diaper 1 in the unfolded state shown in Fig. 2A with the substantially central position CL in the vertical direction as the folding position, and connecting the front side member 11 and the back side member 12 in each lateral end portion 1se that is each end portion in a lateral direction.

As shown in Fig. 2A and Fig. 2B, the diaper 1 in the unfolded state includes a "vertical direction", a "lateral direction" that intersects with the vertical direction, and a "thickness direction" that intersects with the vertical direction and the lateral direction. In respect to the vertical direction, because one side is positioned to the front of the wearer, and the other side is positioned to the back of the wearer, the one side is referred to as a "front side" and the other side is referred to as a "back side". Further, line C-C in Fig. 2A shows the center in the lateral direction.

The planar shape of the front side member 11 and the back side member 12 is substantially rectangular, and the front side member 11 and the back side member 12 are arranged to each of an upper side and a lower side in the vertical direction of the absorbent main body 13, and the skin-side member 16 positioned to a skin side of a wearer and the non-skin-side member 17 positioned to a non-skin side of a wearer are overlaid in the thickness direction. The skin-side member 16 and the non-skin-side member 17 are each a flexible sheet member having elasticity and are formed by, for example, a nonwoven fabric and the like.

Below, the skin-side member 16, the non-skin-side member 17 in a state overlaid on the skin-side member 16, and a plurality of elastic strings 14 arranged along the lateral direction between the skin-side member 16 and the non-skin-side member 17 together are referred to also as an exterior member 10. Here, the plurality of the elastic strings 14 are fixed to the skin-side member 16 and the non-skin-side member 17 with an adhesive such as a hot melt adhesive, elasticity is added to the waist opening 1HB, and the diaper 1 is fitted to the waist of a wearer. The exterior member 10 is made by cutting a composite sheet 20 (to be described later) into a predetermined size.

In the case that the exterior member 10 has been stretched to a state where there is no slack in the non-skin-side member 17, the skin-side member 16 has slack. In other words, when the exterior member 10 is separated to each of the skin-side member 16, the non-skin-side member 17, and the elastic strings 14, the length of the skin-side member 16 in the lateral direction is longer than the length of the non-skin-side member 17 in the lateral direction. In this way, the skin-side member 16 positioned to the skin side of the exterior member 10 is formed with more creases, thus has a softer touch. At this time, when the length of the skin-side member 16 in the lateral direction is excessively long, the appearance becomes unattractive or the touch may instead deteriorate. Thus, in the case where the non-skin-side member 17 is 100 mm, the skin-side member 16 is 103 mm to 115 mm (more preferably, 105 mm to 110 mm). In this way, creases in the skin-side member 16 are not excessively formed, and a diaper 1 with appropriate creases and a soft touch can be formed.

A plurality of crimped parts (processed part) 15 are formed in the diaper 1. The crimped parts 15 are formed in a substantially rectangular shape, and are sections that fix the skin-side member 16 and the non-skin-side member 17 by applying heat and pressure, and the crimped parts 15 restrict movement of the elastic strings 14 in the vertical direction and decrease positional displacement of the elastic strings 14. The crimped parts 15 are formed between adjacent elastic strings 14, and are preferably provided in substantially the central position of the interval between the adjacent elastic strings 14. When the crimped region of the crimped parts 15 becomes large, the exterior member 10 of the diaper 1 becomes hard, and may cause discomfort to a wearer. Thus, the size of the crimped parts is preferably approximately 4 to 9 mm². In Fig. 2B, the crimped parts 15 are shown as flat in the thickness direction, but in actuality, in the thickness direction of the crimped parts 15, the crimped parts 15 become thinner from both sides in the thickness direction (the skin side and the non-skin side), and the crimped parts 15 are shaped squashed from both sides in the thickness direction.

In this embodiment, the crimped parts 15 are to be formed as processed parts, but it is not limited to this. The processed parts may be formed with slit portions made by cutting the exterior member with a cutter, compressed parts made by applying pressure, or welded parts joined by melting a part of the exterior member 10 by applying pressure and heat. In compression or welding, by applying heat at equal to or greater than a certain temperature, a part of the exterior member 10 can be melted to form hole portions that are holes in the exterior member and to improve air permeability of the diaper 1.

The absorbent main body 13 is a substantially rectangular shape in planar view, and is arranged in the center in the lateral direction having the longitudinal direction along the vertical direction of the diaper 1. The shape of the absorbent main body 13 does not have to be a rectangle as shown in Fig. 2A, and for example, may be substantially an hourglass shape. The absorbent main body 13 includes an absorbent body 131 that absorbs and retains a liquid, a liquid permeable surface sheet 132 that covers the absorbent body 131 from a wearer's skin side and permeates excrement such as urine, a liquid impermeable back surface sheet 133 such as a film that covers the absorbent body 131 from the non-skin side and prevents leakage of a liquid from the non-skin side, and a plurality of elastic strings 134 to form leg gather portions LG. The absorbent body 131 is made by molding liquid absorbent fibers such as pulp fibers into a predetermined shape such as a substantially rectangular parallelepiped, and is mixed with high-absorbent polymers inside. The absorbent body 131 may be covered with tissue paper. Barrier cuff portions and the like that prevent leakage to the sides may be provided to both end edges in the lateral direction of the absorbent main body 13.

The pull-on diaper 1 such as shown in Fig. 1 is formed by folding the diaper 1 in the planar state as shown in Fig. 2A with the substantially central position CL in the vertical direction as the folding line, and welding and joining (heat sealing) the lateral end portions 1se of the front side member 11 and the lateral end portions 1se of the back side member 12. The joined lateral end portions 1se, 1se form the end portions 1s in the lateral direction of the diaper 1.

### <Apparatus for manufacturing Composite sheet 20>

Hereinbelow, the apparatus for manufacturing a composite sheet 20 that forms the exterior member 10 of the diaper 1 in this embodiment is described.

In the below description, a direction in which the materials continue is referred to as an "MD direction" (direction of transport), and a width direction of the materials is referred to as a "CD direction". At this time, the thickness direction of the materials, the MD direction which is the direction of continuation, and the CD direction which is the width direction are in an orthogonal relation with each other. The "materials" refer to each of a first continuous sheet 21, a second continuous sheet 22, and an elastic string 23.

The composite sheet 20 is a continuous sheet arranged with elastic strings 23 between the first continuous sheet 21 and the second continuous sheet 22, and is transported in the MD direction in a continuous form, and after various other materials such as the absorbent body 13 are arranged, joined and the like, separated into product shapes, to become the exterior member 10. The first continuous sheet 21 is continuous with a plurality of the skin-side member 16 in a connected state in the lateral direction, the second continuous sheet 22 is continuous with a plurality of the non-skin-side members 17 in a connected state in the lateral direction, and the elastic strings 23 are continuous with a plurality of the elastic strings 14 in a connected state in the lateral direction.

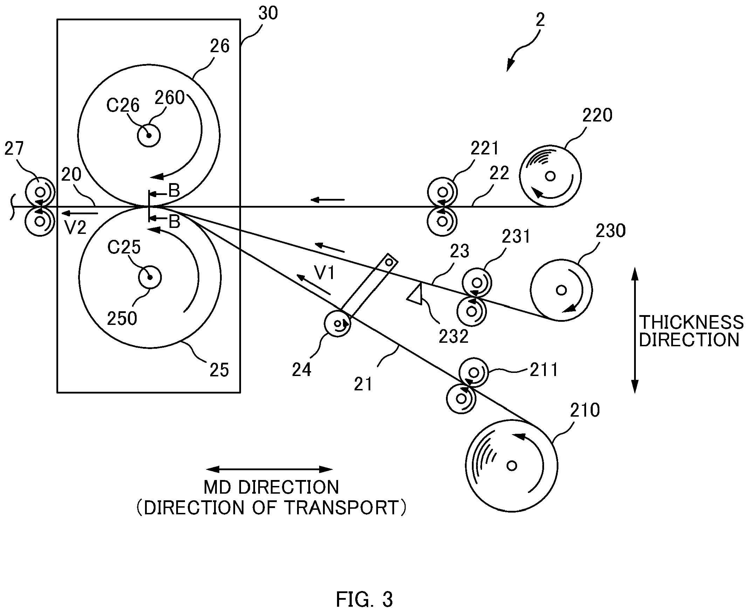

Fig. 3 is a schematic view describing an apparatus 2 for manufacturing a composite sheet 20 in the first embodiment. As shown in Fig. 3, the composite sheet 20 is formed with an apparatus 2 including a first roll 210 to which the first continuous sheet 21 is wrapped around and retained, a second roll 220 to which the second continuous sheet 22 is wrapped around and retained, an elastic string roll 230 to which the elastic string 23 is wrapped around and retained, drive rollers 211 that transports the first continuous sheet21, drive rollers 221 that transport the second continuous sheet 22, drive rollers 231 that transport the elastic string 23, an adhesive applying part 232 that applies an adhesive 2 to the elastic string 23, a lifting part 24 that lifts up the first continuous sheet 21 and the elastic string 23 to the upper side in the thickness direction, a processing device 30 that processes each of the continuous sheets 21, 22, and a drive roller 27 that transports the processed composite sheet 20. Each of the drive rollers 211, 221, 231 drivingly rotates with an appropriate drive source such as a motor. By controlling the rotation of each of the drive rollers 211, 221, 231, transport speed of the materials 21, 22, 23 is adjusted. In Fig. 3, protruding parts including the first protruding parts 251 and the second protruding parts 252 of a process 25 are omitted.

In this embodiment, the drive rollers 211 that transport the first continuous sheet 21 positioned to the lowest side in the thickness direction are preferably heating rollers integrated with a predetermined heat source. By using the heatable drive rollers 211, the first continuous sheet 21 can be heated.

The adhesive applying part 232 that applies an adhesive to the elastic string 23 from the lower side in the thickness direction is preferably provided to the downstream side than the drive rollers 231 and to the upstream side than the processing device 30 and the lifting part 24, in the MD direction. In this way, the adhesive can be applied to the elastic string 23.

The processing device 30 performs crimping to the first continuous sheet 21 and the second continuous sheet 22 from above and below in the thickness direction, while being transported in the MD direction using two rotation bodies. The processing device includes a process roller 25 that is a first rotation body, and an anvil roller 26 that is a second rotation body. The process roller 25 is a rotation body including a plurality of protruding parts 251, 252 (to be described later). The anvil roller 26 is a drum-shaped rotation body, provided to an upper side of the process roller 25 in the thickness direction. The process roller 25 and the anvil roller 26 are each arranged such that an axial direction of a rotation shaft 250 and an axial direction of a rotation shaft 260 are in the CD direction and an outer circumferential surface of each roller are opposed to each other. The process roller 25 and the anvil roller 26 are driven to rotate in the circumferential direction around an axis C25, an axis C26, with an appropriate drive source such as a motor. In this embodiment, the process roller 25 is incorporated with a heat source, and tip end portions of the first protruding parts 251 and the second protruding parts 252 heat the first continuous sheet 21. When the first continuous sheet 21, the elastic string 23, and the second continuous sheet 22 are passed through a space between rolls of the process roller 25 and the anvil roller 26 which are driven to be rotated, the first continuous sheet 21 and the second continuous sheet 22 are crimped to form the crimped parts 15.

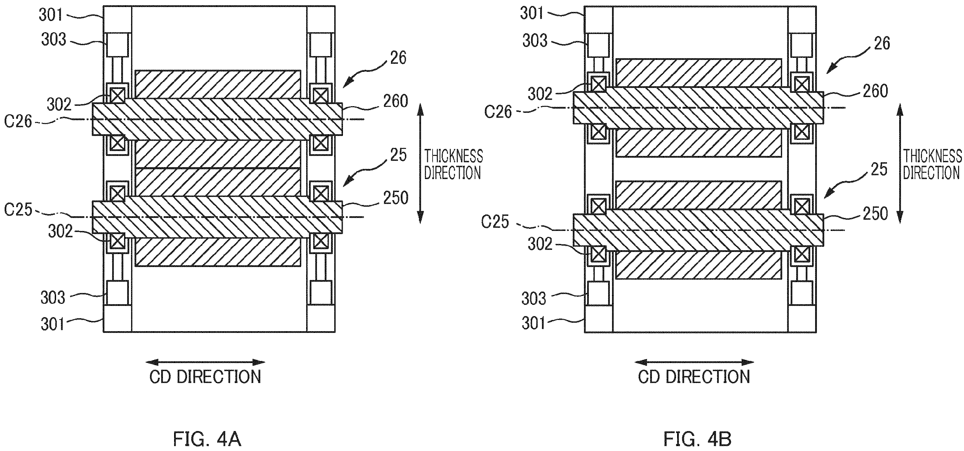

Fig. 4A is a schematic central sectional view describing the processing device 30 during processing. In Fig. 4A, the protruding parts 251, 252 of the process roller 25 are omitted. As shown in Fig. 4A, the processing device 30 includes a housing 301 to support the process roller 25 and the anvil roller 26, in both side in the CD direction. The housing 301 is, for example, a rectangular frame body, and both end portions of the rotation shaft 250 of the process roller 25 and both end portions of the anvil roller 26 are arranged inside the housing. Each of the end portions of the rotation shafts 250, 260 are rotatably supported with a bearing 302, which rotatably supports each of the process roller 25 and the anvil roller 26.

It is preferable to include an actuator 303 such as a hydraulic cylinder between a housing 301 and a bearing 302 and to configure each of the process roller 25 and the anvil roller 26 to be able to be raised and lowered.

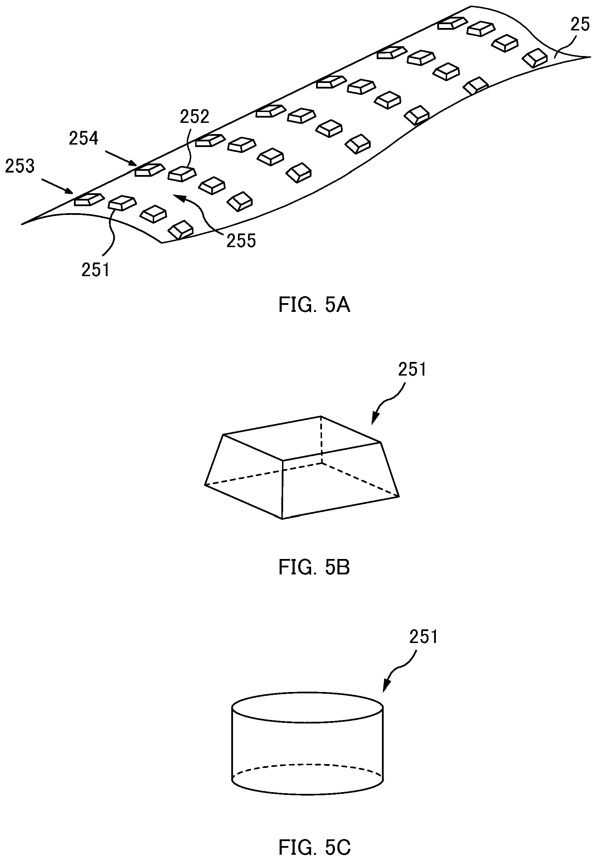

Fig. 5A is a view showing a part of an outer peripheral surface of a process roller 25 of a first embodiment. Fig. 5B is a schematic perspective view of one first protruding part 251 in Fig. 5A. Fig. 5C is a schematic perspective view of one modification of a first protruding part 251.

As shown in Fig. 5A, the process roller 25 includes the first protruding parts 251a and the second protruding parts 252 protruding radially outwardly on the circumferential surface. Between the first protruding parts 251a and the second protruding parts 252 are provided recessed parts 255 that are parts more recessed than the first protruding parts 251a and the second protruding parts 252.

The shape of the first protruding part 251 is a substantially quadrangular prism, as shown in Fig. 5B, and a planar area to the outermost side in the radial direction is a smaller shape than a planar area to the innermost side in the radial direction.

In this embodiment, the shape of the first protruding part 251 and the second protruding part 252 is shown as a substantially quadrangular prism, and the shape of the first protruding part 251 and the second protruding part 252 can be changed arbitrarily. As shown in Fig. 5C, the shape protruding parts can be a column, or may be other arbitrary shapes such as a triangular prism.

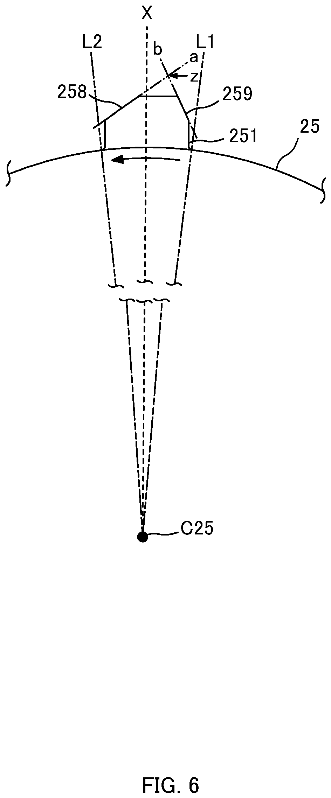

As shown in Fig. 6, an end part to an outer side in the radial direction of the first protruding part 251 may be formed tapered in an arbitrary shape. Fig. 6 is a schematic view of one modification of a first protruding part 251 as viewed from an axial direction of the rotation shaft 250. As shown in Fig. 6, when viewed from an axial direction of the rotation shaft 250, the process roller 25 rotates anticlockwise. The first protruding part 251 is provided within a predetermined angle range θ. More specifically, as shown in Fig. 6, the first protruding part 251 is provided in a region sandwiched between two lines L1, L2 that each passes through the rotational center (axis C25) of the process roller 25. Line X is a line showing the center of a predetermined angle range θ.

The first protruding part 251 includes a first inclined surface 258 and a second inclined surface 259 in the tip end portion in the protruding direction. The first inclined surface 258 is provided to a downstream side in the direction of rotation, and the second inclined surface 259 is provided to an upstream side in the direction of rotation. When an assumed plane including the first inclined surface 258 is referred to as a first plane "a" and an assumed plane including the second inclined surface 259 is referred to as a second plane "b", a line of intersection "z" which is a position where the first plane "a" and the second plane "b" intersect each other in the direction of rotation of the process roller 25 is positioned to the upstream side than the central position of the first protruding part 251, and in Fig. 6, the line of intersection "z" is positioned to the upstream side than the line X. In other words, the first plane "a" which is a plane extending from the first inclined surface 258 is an inclined surface less inclined than the second plane "b" which is a plane extending from the second inclined surface 259, and the length of the inclined surface of the first plane "a" is longer than the length of the inclined surface of the second plane "b".

When using such a first protruding part 251, initially the first inclined surface 258 of the first protruding part 251 contacts the first continuous sheet 21, with the rotation of the process roller 25, and then the second inclined surface 259 contacts the first continuous sheet 21. In this way, as the first inclined surface "a" having a relatively gentle inclined surface contacts the anvil roller 26 via the first continuous sheet 21 and the like, the impact of when contact starts is alleviated, and the possibility of breakage of the first protruding part 251 due to contact can be decreased. When the first protruding part 251 moves away from the first continuous sheet 21, the second inclined surface "b" having a relatively steep slope moves away quickly from the first continuous sheet 21, thus the possibility of excessive processing or breakage of materials of the first continuous sheet 21 can be decreased.

In Fig. 5B, Fig. 5C, and Fig. 6, the first protruding part 251 is shown, and the second protruding part 252 may also be a similar shape, and the first protruding part 251 or the second protruding part 252 may each have a different shape from each other.

As shown in Fig. 5A, the process roller 25 includes a first protruding part group 253 made of a plurality of the first protruding parts along the circumferential direction and a second protruding part group 254 made of a plurality of the second protruding part 252 along the circumferential direction.

The drive rollers 27 are provided downstream in the MD direction of the processing device 30, and the drive rollers 27 transport the composite sheet 20, and then processing such as cutting into a predetermined shape is performed. These drive rollers 27 are driven and rotated with an appropriate drive source such as a motor similarly to the drive rollers 221, 223, and this rotation is controlled to adjust the transport speed of the composite sheet 20.

### <Method for manufacturing Composite Sheet 20>

A method for manufacturing a composite sheet 20 of this embodiment is described below.

As shown in Fig. 3, the first roll 210, the elastic string roll 230, and the second roll 220 are included in order from the lower side in the thickness direction, and the first continuous sheet 21, the elastic string 23, and the second continuous sheet 22 are each transported to the processing device 30 with the drive rollers 211, 221, 223. Here, the first continuous sheet 21, the second continuous sheet 22, and the elastic string 23 are transported in a state added with a certain amount of tension.

While transporting, by using the drive rollers 211 which also serve as heat rollers, the first continuous sheet 21 can be heated. When the first continuous sheet 21 is heated to a predetermined temperature, the sheet member such as a nonwoven fabric becomes soft due to heating, thus the first continuous sheet 21 can also be made soft. When the first continuous sheet 21 becomes soft, the first sheet 21 can easily be made to follow the recessed and protruding shapes of the first protruding parts 251 and the second protruding parts 252 of the process roller 25 to be described later, thus the elastic string 23 can easily be placed in the recessed parts 255 between the first protruding parts 251 and the second protruding parts 252, and the positional displacement of the elastic string 23 can be decreased.

Heating using the drive rollers 211 is preheating to heat in advance before processing of crimping, compressing and the like with the processing device 30, and with this preheating, the possibility of excessively damaging the first continuous sheet 21 with rapid heating during processing can be decreased.

This preheating is to be performed at a temperature higher than normal temperature, and lower than the temperature during processing with the processing device 30, and more preferably, when a melting point of such as a nonwoven fabric configuring the first continuous sheet 21 is "Tm", the first continuous sheet 21 may be heated in a range of (Tm - 60°C) - (Tm + 10°C).

During transporting of the elastic string 23, an adhesive is applied to the elastic string 23 with the adhesive applying part 232, the first continuous sheet 21, the elastic string 23, and the second continuous sheet 22 can be adhered with the adhesive applied to the elastic string 23 positioned between the sheets 21, 22, thus being able to form a softer composite sheet 20 than when the adhesive is directly applied to the first continuous sheet 21 and the second continuous sheet 22 and adhered.

The materials 21, 22, 23 transported with the drive rollers 211, 221, 231 are overlaid in order of the first continuous sheet 21, the elastic string 23, and the second continuous sheet 22, from the lower side in the thickness direction, on the process roller 25 of the processing device 30. At this time, the first continuous sheet 21 is transported on the process roller 25 to follow the recessed and protruding shapes of the first protruding part 251, the second protruding part 252, and the recessed portion 255 of the process roller 25. The elastic string 23 is transported so as to be placed in the recessed part 255 between the first protruding part 251 and the second protruding part 252. The second continuous sheet 22 is transported in a state such that it can be placed on the tip ends of the first protruding parts 251 and the second protruding parts 252. The materials 21, 22, 23 arranged on the process roller 25 are transported to a position in which the anvil roller 26 and the first protruding part 251 and the second protruding part 252 of the process roller 25 come into contact, on the process roller 25.

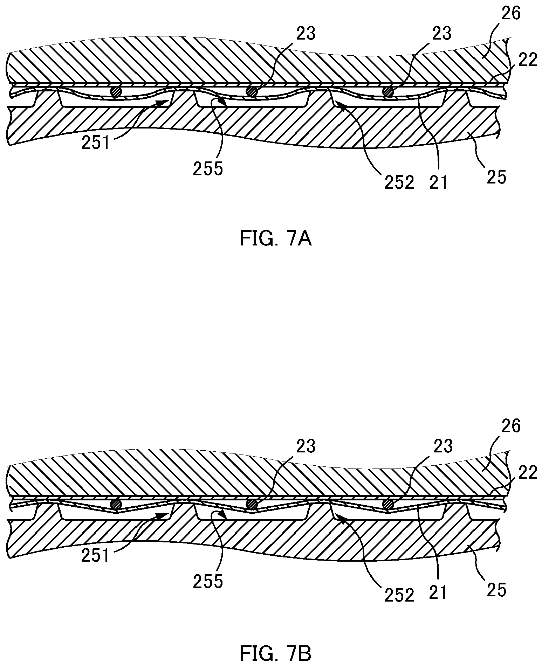

Fig. 7A is a view along arrows B-B in Fig. 3. While the process roller 25 rotates, the anvil roller 26 rotates in an opposite direction, and, as shown in Fig. 7A, the first protruding part 251 and the second protruding part 252 of the process roller 25 come into contact with the anvil roller 26 via the first continuous sheet 21 and the second continuous sheet 22, and thus pressure and heat is applied to the first continuous sheet 21 and the second continuous sheet 22. In this way, the crimped parts 15 are formed in parts that came in contact with the tip end portions of the first protruding parts 251 and the second protruding parts 252, and the composite sheet 20 is formed. At this time, by heating the first continuous sheet 21 and the second continuous sheet 22 to a range of (Tm - 60°C) - (Tm + 30°C) with the heat source integrated in the process roller 25, the crimped parts 15 made by joining the first continuous sheet 21 and the second continuous sheet 22 can be formed. At this time, when preheating is performed with the drive rollers 211 in advance, heating with the process roller 25 can gently raise the temperature of the first continuous sheet 21 and the second continuous sheet 22, thus decreasing damage to the first continuous sheet 21 and the second continuous sheet 22.

Here, as shown in Fig. 7A, the first continuous sheet 21 that is positioned nearest to the process roller 25 side is curved according to the recessed and protruding shapes of the first protruding parts 251, the second protruding parts 252, and the recessed parts 255. Further, the lower half of the elastic strings 23 positioned to the upper side in the thickness direction of the first continuous sheet 21 is placed in the recessed parts 255 between the first protruding parts 251 and the second protruding parts 252.

In this way, by forming the crimped parts 15 with the elastic strings 23 placed in the recessed parts 255 between the first protruding part 251 and the second protruding part 252, the possibility of the elastic strings 23 erroneously coming in contact with each of the tip end portions of the first protruding part 251 and the second protruding part 252 can be decreased and the possibility of the elastic strings 23 being cut can be decreased.

In this embodiment, as similar to the case where the lower half of the elastic strings 23 are placed in the recessed parts 255, the elastic strings 23 may be partially placed in the recessed parts 255, or the elastic strings 23 may be entirely placed in the recessed parts 255.

For example, the first continuous sheet 21 may be curved along the recessed and protruding shapes of the protruding parts 251, 252, and the recessed parts 255, and the first continuous sheet 21 may be made to contact the most recessed part of the recessed parts 255, and the elastic strings 23 may be entirely placed in the recessed parts 255. By curving the first continuous sheet 21 to contact the most recessed part of the recessed parts 255, the elastic strings 23 can be more easily placed in the recessed parts 255, and the possibility of erroneously cutting the elastic strings 23 with the processing device 30 can be decreased. In this case, the adhesive may adhere to the process roller 25, thus regular cleaning is needed.

Crimping is more preferably performed by transporting on the process roller 25 the first continuous sheet 21 positioned to the process roller 25 side, with the first continuous sheet 21 in a state separated by a predetermined distance from the most recessed part of the recessed parts 255. In other words, the first continuous sheet 21 is located to not contact the innermost side in the radial direction of the recessed parts 255. In this way, the possibility of the adhesive applied to the elastic strings 23 adhering to the process roller 25 can be decreased, and the burden of maintenance and the like of the apparatus 2 can be decreased. This applies not just for the case where the adhesive has been applied to the elastic strings 23, and also for the case where the adhesive has been applied to either the first continuous sheet 21 or the second continuous sheet 22, the possibility of the adhesive adhering to the process roller 25 can be decreased.

At this time, the first continuous sheet 21 positioned to the process roller 25 side may be slack as shown in Fig. 7A, or may in a state with no slack between the first protruding part 251 and the second protruding part 252 as shown in Fig. 7B. Fig. 7B is a view taken along arrows B-B in Fig. 3 of a modified example. In either the case where the first continuous sheet 21 positioned between the first protruding part 251 and the second protruding part 252 is slack as shown in Fig. 7A, and the case where the first continuous sheet 21 positioned between the first protruding part 251 and the second protruding part 252 is not slack as shown in Fig. 7B, the length of the first continuous sheet 21 positioned between the first protruding part 251 and the second protruding part 252 is longer than the length of the second continuous sheet 22. Thus, in the case where the processed composite sheet 20 is extended in the continuous direction, and the second continuous sheet 22 has no slack, the first continuous sheet 21 has slack. In other words, when creases of the second continuous sheet 22 are stretched completely, the first continuous sheet 21 includes creases. Thus, the first continuous sheet 21 of the composite sheet 20 is formed with more creases than the second continuous sheet 22, and becomes softer than the first continuous sheet 21.

At this time, by making the proportion of the length of the first continuous sheet 21 to be 103 mm - 115 mm (more preferably 105 mm - 110 mm), in respect to the unit length 100 mm of the second continuous sheet 22, an appropriate amount of creases can be formed in the first continuous sheet 21. In this way, the first continuous sheet 21 and the second continuous sheet 22 can each be stably transported, and excessive creases can be prevented from being formed in the first continuous sheet 21 and the possibility of erroneously cutting the elastic string 23 can be decreased. Further, with an appropriate amount of creases in the first continuous sheet 21, the skin-side member 16 can be formed softer, to be able to provide an absorbent article with good touch to users.

The composite sheet 20 formed with the crimped parts 15 with the processing device 30 is transported to a predetermined location with the drive rollers 27. At this time, transport speed (v2) of the composite sheet 20 transported with the drive rollers 27 is preferably faster than transport speed (v1) of the first continuous sheet 21 transported with the drive rollers 211 (v2>v1). In other words, from the part where the protruding parts 251, 252 of the process roller 25 and the anvil roller 26 come into contact, the transport speed (v1) to the upstream side in the MD direction is made relatively slow, and the transport speed (v2) to the downstream side in the MD direction is made relatively fast. For example, in the case where the roll diameter of the drive roller 27 and the drive roller 211 are the same, the number of rotation per unit time of the drive roller 27 may be made greater than that of the drive roller 211. In this way, while the first continuous sheet 21 is being transported to the processing device 30, the first continuous sheet 21 is transported at a relatively slow speed (v1), thus the first continuous sheet 21 easily follows the recessed and protruding shapes of the protruding parts 251, 252 and the recessed parts 255 of the process roller 25, and the elastic strings 23 can be easily placed in the recessed parts 255. On the other hand, by making the transport speed (v2) of the drive rollers 21 to the downstream side in the MD direction than the processing device 30 relatively fast, the composite sheet 20 can be made to a state that is stretched more, namely tensioned, than before the processing, to decrease positional displacement caused by transporting, and to be able to transport efficiently.

When the composite sheet 20 is formed by processing with the processing device 30, the composite sheet 20 is transported to a predetermined position with the drive rollers 27, and cut and the like to a predetermined size.

### <Case where manufacturing of Composite Sheet 20 is stopped>

Next, a case where processing with the processing device 30 is stopped and transporting of the materials 21, 22, 23 is stopped is described.

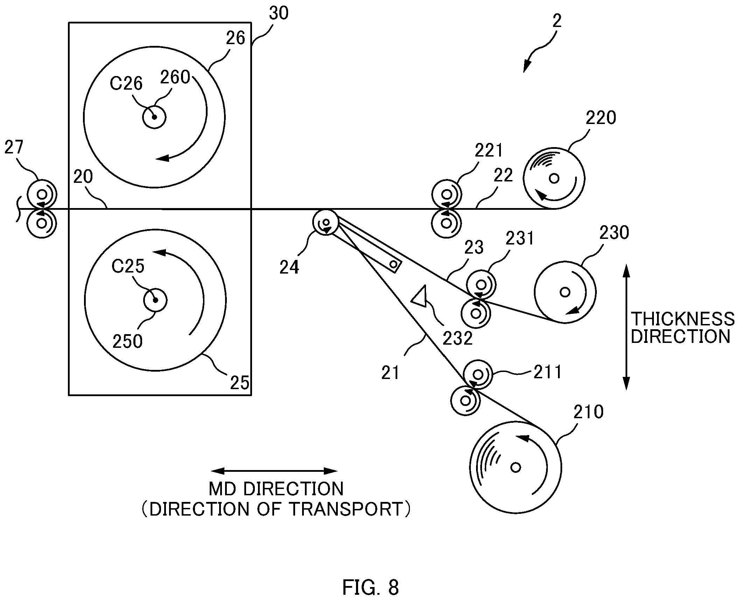

In the case where processing with the processing device 30 is stopped and transporting of the materials 21, 22, and 23 is stopped for some reason, there is a possibility that heating with the first protruding parts 251 and the second protruding parts 252 will continue to the first continuous sheet 21 and the second continuous sheet. For this reason, the first continuous sheet 21 positioned to the process roller 25 side is placed away from the process roller 25. For example, as shown in Fig. 8, preferably, the process roller 25 and the anvil roller 26 are placed apart, and the first continuous sheet 21 and the elastic string 23 are lifted to the upper side in the thickness direction with the lifting part 24, to prevent the first continuous sheet 21 and the second continuous sheet 22 from being heated continuously. Fig. 4B is a schematic central sectional view describing the processing device 30 that has stopped processing. Fig. 8 is a schematic view describing the apparatus 2 that has stopped processing. In Fig. 4B, the protruding parts 251, 252 of the process roller 25 have been omitted.

When process with the processing device 30 and each of the drive rollers 221, 231, 241, 27 are stopped, first, the apparatus 2 lowers the process roller 25 to the lower side in the thickness direction, as shown in Fig. 4B and Fig. 8, and raises the anvil roller 26 to the upper side in the thickness direction. In this embodiment, the process roller 25 is lowered and the anvil roller 26 is raised using an actuator 303. One of the upper or lower actuators 303 may be omitted and either one of the process roller 25 or the anvil roller 26 can be made to move up and down. In this way, the first continuous sheet 21, the second continuous sheet 22, and the elastic string 23 positioned in a space between the process roller 25 and the anvil roller 26 in the processing device 30 can be placed apart from the process roller 25.

Next, the first continuous sheet 21 and the elastic string 23 are lifted up to the upper side in the thickness direction with rotation of the lifting part 24. At this time, the first continuous sheet 21 and the elastic string 23 are lifted up to the same height as the second continuous sheet 22 in the thickness direction. The first continuous sheet 21 and the elastic string 23 are transported on the process roller 25 with the first continuous sheet 21 following the recessed and protruding shapes of the first protruding parts 251, the second protruding parts, and with the elastic string 23 placed in the recessed parts 255. Thus, with just the raising and lowering of the process roller 25 and the anvil roller 26, the first continuous sheet 21 and the elastic string are still placed in the recesses and protrusions on the process roller 25 and there is a possibility that the first continuous sheet 21 and the elastic string 23 cannot be sufficiently placed apart from the process roller 25. For this reason, by using the lifting part 24, the first continuous sheet 21 and the elastic string 23 can be more certainly placed apart from the process roller 25.

### ===Other Embodiments===

The embodiment of this invention has been described above, and the above embodiment is to facilitate understanding of this invention, and does not limit understanding of this invention. Further, this invention may be changed or modified. For example, the below modification is possible.

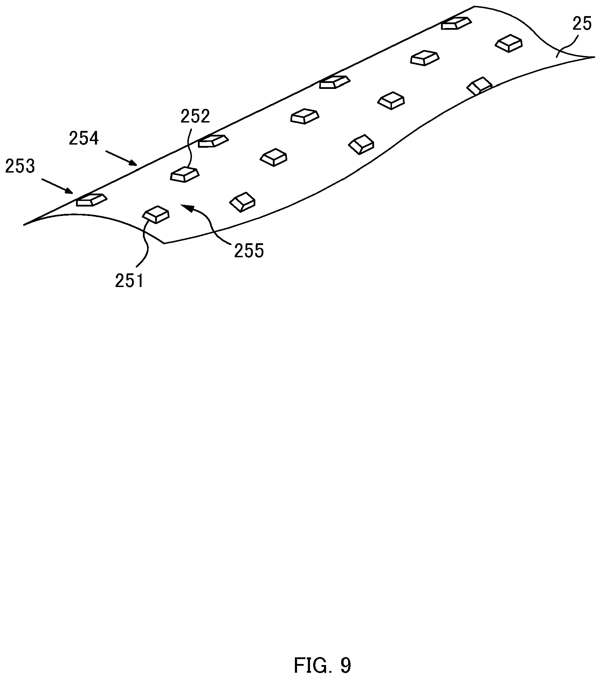

In the first embodiment, the first protruding parts 251 and the second protruding parts 252 of the first protruding part group 253 and the second protruding part group 254 of the process roller 25, are arranged in the same position in the axial direction and the circumferential direction, but it is not limited to this. Fig. 9 is a view showing a part of the outer circumferential surface of the process roller 25 of other embodiments. As shown in Fig. 9, the protruding parts 251 of the first protruding part group 253, and the protruding parts 252 of the second protruding part group 254 are arranged in positions different from each other in the axial direction and the circumferential direction. In other words, the protruding parts are arranged in namely a staggered shape. By using the process roller 25 having these protruding parts 251, 252, movement of the elastic string 23 during processing can be restricted, and the possibility of the elastic string 23 being erroneously cut can be decreased.

The number and arrangement of the first protruding parts 251 and the second protruding parts 252 can be decided arbitrarily separately. In the above-described embodiment, a plurality of the first protruding parts 251 and a plurality of the second protruding parts 252 are arranged, but it is not limited to this. The process roller 25 may have at least one first protruding part 251 and at least one second protruding part 252. Further, the first protruding part 251 and the second protruding part 252 may be ribs formed continuously along the circumferential surface of the process roller 25, and the ribs may be provided with an interval in the axial direction. In such a case, the movement of the elastic string 23 can be restricted during processing, to be able to decrease the possibility of the elastic string 23 being cut erroneously.

Using the process roller 25 of the first rotation body and the anvil roller 26 of the second rotation body, hole portions may be formed. Holes can be formed in the first continuous sheet 21 and the second continuous sheet 22 by making the heating temperature of the process roller 25 to be (Tm + 5°C) - (Tm + 40°C).

As the second rotation body, the anvil roller 26 or the cutter roller 28 or the like, and various rotation bodies according to the aim of the processing can be used.

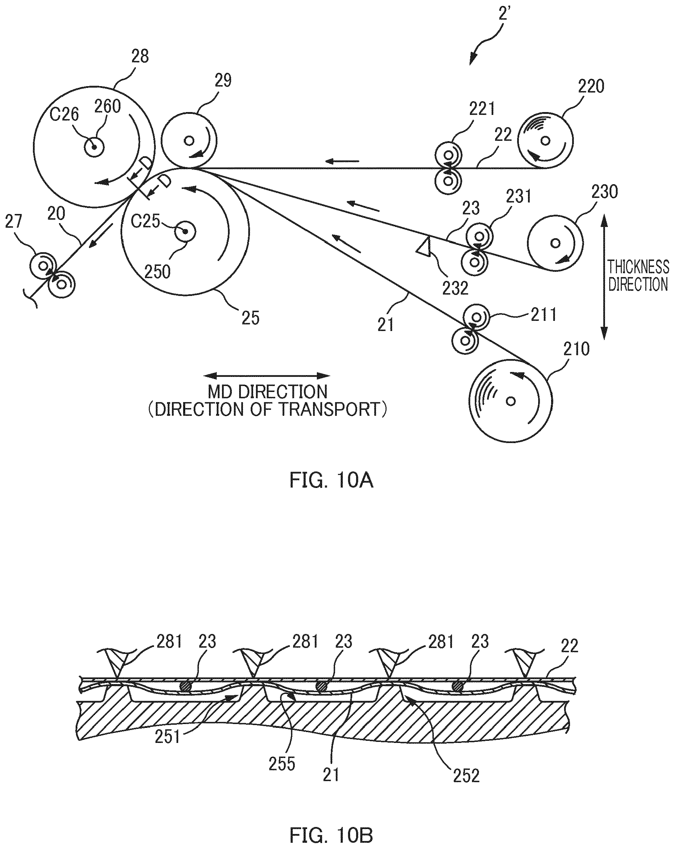

In the above-described embodiment, crimping is performed using the process roller 25 as the first rotation body and the anvil roller 26 as the second rotation body, but it is not limited to this. The second rotation body may perform processing to form holes and the like to the first continuous sheet 21 and the second continuous sheet 22, using not the anvil roller 26 but the cutter roll 28. Fig. 10A is a diagram describing apparatus 2' for manufacturing a composite sheet 20 of another embodiment. Fig. 10B is a view taken along arrows D-D in Fig. 10A.

As shown in Fig. 10A, the apparatus 2' overlays in order from the bottom on a process roller 25, a first continuous sheet 21 transported from a first roll 210, an elastic string 23 transported from an elastic string roll 230, a second continuous sheet 22 transported from a second roll 220, passes through the above between the process roller 25 and an anvil roller 29, then using the process roller 25 and a cutter roller 28 forms holes in a first continuous sheet 21 and a second continuous sheet 22.

Before processing with the cutter roller 28, the first continuous sheet 21 and the elastic string 23 and the second continuous sheet 22 are passed between the process roller 25 and the anvil roller 29 to be sandwiched, so that at least the elastic string 23 can be partially easily put in the recessed parts 255 between the first protruding parts 251 and the second protruding parts 252.

Next, as shown in Fig. 10B, holes are formed with the process roller 25 and the cutter roller 28. The cutter roller 28 has cutter parts 281 in a tip end portion in an outer periphery in the radial direction. While the process roller 25 rotates, the cutter roller 28 rotates in an opposite direction, thus the cutter parts 281 and the first protruding parts 251 and the second protruding parts 252 are in a positional relation periodically opposing each other. At this time, the holes are formed with the cutter parts 281, with the elastic strings 23 more certainly placed in the recessed parts 255, thus the possibility of erroneously processing the elastic string 23 can be further decreased.

In the above-described embodiment, the adhesive is applied to the elastic string 23, but the adhesive does not have to be applied. Moving of the elastic string 23 can be restricted with crimped parts 15, and the possibility of the elastic string 23 moving can be decreased, and further a softer exterior member 10 can be provided.

In the above-described embodiment, in the case that manufacturing of the composite sheet 20 is stopped, after stopping the processing and the transporting, the first continuous sheet 21 and the elastic string 23 are lifted up using the lifting part 24, and the process roller 25 and the anvil roller 26 in the processing device 30 are made to move up and down, but it is not limited to the above. Before stopping the processing and the transporting the process roller 25 and the anvil roller 26 in the processing device 30 may be made to move up and down, and the anvil roller 26 may be made to move up and down using the lifting part 24. Specifically, at the time the apparatus 2 receives a signal to stop the processing and the transporting, the lifting part 24 may lift up the first continuous sheet 21 and the elastic string 23, and the process roller 25 and the anvil roller 26 may be made to move up and down.

In the above-described embodiment, in the case where the second continuous sheet 22 is stretched until there is no slack, the first continuous sheet 21 is made to have slack, such that the skin side member 16 is formed with more creases than the non-skin side member 17, but it is not limited to the above. In the case where the first continuous sheet 21 is stretched until there is no slack, the second continuous sheet 22 may be configured to have slack. In this way, the non-skin side member 17 will be formed with more creases than the skin side member 16, thus being able to give a softer impression visually.

Further, in the above-described embodiment, namely a 3-piece type disposable diaper 1 is illustrated as an example of the absorbent article, but it is not limited to this in any way. For example, a 2-piece type disposable diaper including an exterior sheet having a front side part, a crotch part, and a back side part as a first component, and an absorbent main body to be fixed to a skin side surface of an exterior sheet as a second component, or a tape-type disposable diaper may be manufactured.

### Reference Signs List

1 diaper (disposable diaper),
1HB waist opening part, 1HL leg opening part,
1se lateral end portion, 1s end portion,
10 exterior member, 11 front member, 12 back member,
13 absorbent main body,
131 absorbent body, 132 surface sheet,
133 back surface sheet, 134 elastic string,
14 elastic string, 141 elastic string,
15 crimped part (processed part),
16 skin-side member, 17 non-skin-side member,
2, 2' apparatus,
20 composite sheet,
21 first continuous sheet, 210 first roll,
211 drive roller,
22 second continuous sheet, 220 second roll,
221 drive roller,
23 elastic string, 230 elastic string roll,
231 drive roller, 232 adhesive applying portion,
24 lifting part,
25 process roller (first rotation body),
251 first protruding part, 252 second protruding part,
253 first protruding part group, 254 second protruding part group,
255 recessed part,
258 first inclined surface, 259 second inclined surface,
26 anvil roller (second rotation body),
27 drive roller,
28 cutter roller, 281 cutter part,
29 anvil roller,
30 processing device,
301 housing, 302 bearing, 303actuator

## Claims

1. A method for manufacturing a composite sheet (20) of an absorbent article (1) to manufacture a composite sheet including a first continuous sheet (21), a second continuous sheet (22), and an elastic string (23) to be arranged between the first continuous sheet and the second continuous sheet, the method for manufacturing a composite sheet of an absorbent article comprising:
transporting, in a direction of continuation, the first continuous sheet (21), the second continuous sheet (22), and the elastic string (23), being materials, in a state where the elastic string arranged between the first continuous sheet and the second continuous sheet is at least partially interposed between first protruding parts (251) and second protruding parts (252), by causing a rotatable first rotation body (25) to rotate, the first rotation body (25) including the first protruding parts (251) and the second protruding parts (252) protruding radially outwardly; and
performing processing to the first continuous sheet (21) and the second continuous sheet (22) by sandwiching the first continuous sheet and the second continuous sheet between a rotatable second rotation body (26) and the first protruding parts (251) and the second protruding parts (252), during transporting with the first rotation body (25);
wherein
a thickness direction of the materials, the direction of continuation, and a width direction are in an orthogonal relation with each other; and
while rotation of the first rotation body (25) is stopped, the first continuous sheet, positioned to the first rotation body side of the first continuous sheet (21) and the second continuous sheet (22), and the elastic string (23) are placed apart from the first rotation body and lifted up to an upper side in the thickness direction with a lifting part (24).

2. A method for manufacturing a composite sheet of an absorbent article according to claim 1, wherein
during the processing, the first protruding parts (251) and the second protruding parts (252) heat the first continuous sheet (21) and the second continuous sheet (22).

3. A method for manufacturing a composite sheet of an absorbent article according to claim 1 or 2, wherein
while rotation of the first rotation body (25) is stopped, a distance between the first rotation body (25) and the second rotation body (26) is wider than a distance between the first rotation body and the second rotation body during the processing.

4. A method for manufacturing a composite sheet of an absorbent article according to any one of claims 1 to 3, wherein
while rotation of the first rotation body (25) is stopped,
a transfer path located immediately before contacting the first rotation body, of a transfer path of the first continuous sheet positioned to the first rotation body side of the first continuous sheet (21) and the second continuous sheet (22), is in a state changed to a direction apart from the first rotation body.

5. A method for manufacturing a composite sheet of an absorbent article according to any one of claims 1 to 4, wherein
a transport speed to transport the first continuous sheet (21), the second continuous sheet (22), and the elastic string (23) is faster after the processing than before the processing.

6. A method for manufacturing a composite sheet of an absorbent article according to any one of claims 1 to 5, wherein
the elastic string (23) is arranged between the first continuous sheet (21) and the second continuous sheet (22) in a state attached with an adhesive.

7. A method for manufacturing a composite sheet of an absorbent article according to any one of claims 1 to 6, wherein
an adhesive is in a state attached between the elastic string (23) and the first continuous sheet positioned to the first rotation body side of the first continuous sheet (21) and the second continuous sheet (22),
the first rotation body includes recessed parts (255) between the first protruding parts (251) and the second protruding parts (252), and
the first continuous sheet, positioned to the first rotation body side of the first continuous sheet (21) and the second continuous sheet (22), is located apart from a most recessed portion of the recessed parts (255) by a predetermined distance.

8. A method for manufacturing a composite sheet of an absorbent article according to any one of claims 1 to 7, wherein
at least one protruding part of the first protruding part (251) and the second protruding part (252) includes a first inclined surface (258) and a second inclined surface (259) in a tip end portion in a protruding direction,
in a direction of rotation of the first rotation body (25), the first inclined surface (258) is provided to a downstream side than the second inclined surface (259), and
when a plane ("a") including the first inclined surface (258) is a first plane, and
a plane ("b") including the second inclined surface (259) is a second plane,
a position in which the first plane and the second plane intersect, in the direction of rotation, is positioned to an upstream side than a central position of the at least one protruding part.

9. A method for manufacturing a composite sheet of an absorbent article according to any one of claims 1 to 8, wherein
before the processing, the first continuous sheet, positioned to at least the first rotation body side of the first continuous sheet (21) and the second continuous sheet, (22) is heated.

10. A method for manufacturing a composite sheet of an absorbent article according to any one of claims 1 to 9, wherein
the first rotation body (25) includes a first protruding part group (253) formed from a plurality of protruding parts along a circumferential direction and a second protruding part group (254) formed from a plurality of protruding parts along a circumferential direction,
the first protruding part group (253) includes the first protruding parts (251),
the second protruding part group (254) includes the second protruding parts (252), and
each protruding part of the first protruding part group (253) and each protruding part of the second protruding part group (254) are arranged in positions different from each other in an axial direction and the circumferential direction.

11. A method for manufacturing a composite sheet of an absorbent article according to any one of claims 1 to 10, wherein
in a certain position in a direction of rotation of the first rotation body (25),
a plurality of the elastic strings (23) are arranged between the first continuous sheet (21) and the second continuous sheet (22),
the first continuous sheet, the second continuous sheet, and the elastic strings are transported to another position in the direction of rotation using the first rotation body (25), and
processing is performed by sandwiching the first continuous sheet (21) and the second continuous sheet (22) in the other position.

12. An apparatus (2) for manufacturing a composite sheet (20) of an absorbent article (1) to manufacture a composite sheet including a first continuous sheet (21), a second continuous sheet (22), and an elastic string (23) arranged between the first continuous sheet and the second continuous sheet, the apparatus for manufacturing comprising:
a transporting part that transports in a direction of continuation the first continuous sheet (21), the second continuous sheet (22), and the elastic string (23), being materials, in a state where the elastic string arranged between the first continuous sheet and the second continuous sheet is at least partially interposed between first protruding parts (251) and second protruding parts (252), by causing a rotatable first rotation body (25) to rotate, the first rotation body (25) including the first protruding parts (251) and the second protruding parts (252) protruding radially outwardly;
a processing part that processes the first continuous sheet (21) and the second continuous sheet (22) by sandwiching the first continuous sheet and the second continuous sheet between a rotatable second rotation body (26) and the first protruding parts (251) and the second protruding parts (252), during transporting with the first rotation body (25); and
a lifting part (24) that lifts up the first continuous sheet (21) and the elastic string (23) to an upper side in a thickness direction to place them apart from the first rotation body when rotation of the first rotation body is stopped;
wherein the thickness direction of the materials, the direction of continuation, and a width direction are in an orthogonal relation with each other.

## Patentansprüche

1. Verfahren für die Herstellung einer Verbundlage (20) eines absorbierenden Artikels (1), um eine Verbundlage herzustellen, die Folgendes einschließt: eine erste kontinuierliche Lage (21), eine zweite kontinuierliche Lage (22) und ein elastisches Band (23), das zwischen der ersten kontinuierlichen Lage und der zweiten kontinuierlichen Lage anzuordnen ist, wobei das Verfahren für die Herstellung einer Verbundlage eines absorbierenden Artikels Folgendes umfasst:
Fördern in einer fortführenden Richtung der ersten kontinuierlichen Lage (21), der zweiten kontinuierlichen Lage (22) und des elastischen Bands (23), die Materialien sind, in einem Zustand, wo das elastische Band, das zwischen der ersten kontinuierlichen Lage und der zweiten kontinuierlichen Lage angeordnet ist, zumindest teilweise zwischen ersten vorstehenden Teilen (251) und zweiten vorstehenden Teilen (252) durch Bewirken einer Rotation eines rotierbaren ersten Rotationskörpers (25) eingefügt ist, wobei der erste Rotationskörper (25) die ersten vorstehenden Teile (251) und die zweiten vorstehenden Teile (252) einschließt, die radial nach außen vorstehen; und
Durchführen der Verarbeitung mit der ersten kontinuierlichen Lage (21) und der zweiten kontinuierlichen Lage (22) durch Zusammenpressen der ersten kontinuierlichen Lage und der zweiten kontinuierlichen Lage zwischen einem rotierbaren zweiten Rotationskörper (26) und den ersten vorstehenden Teilen (251) und den zweiten vorstehenden Teilen (252) während des Förderns mit dem ersten Rotationskörper (25); wobei
eine Dickenrichtung der Materialien, die fortführende Richtung und eine Breitenrichtung in einer orthogonalen Beziehung zueinander stehen; und,
während die Rotation des ersten Rotationskörpers (25) gestoppt ist, die erste kontinuierliche Lage, die auf der Seite des ersten Rotationskörpers der ersten kontinuierlichen Lage (21) und der zweiten kontinuierlichen Lage (22) positioniert ist, und das elastische Band (23) von dem ersten Rotationskörper getrennt positioniert sind und zu einer oberen Seite in der Dickenrichtung mit einem Hebeteil (24) angehoben werden.

2. Verfahren für die Herstellung einer Verbundlage eines absorbierenden Artikels nach Anspruch 1, wobei
während der Verarbeitung die ersten vorstehenden Teile (251) und die zweiten vorstehenden Teile (252) die erste kontinuierliche Lage (21) und die zweite kontinuierliche Lage (22) erwärmen.

3. Verfahren für die Herstellung einer Verbundlage eines absorbierenden Artikels nach Anspruch 1 oder 2, wobei,
während die Rotation des ersten Rotationskörpers (25) gestoppt ist, ein Abstand zwischen dem ersten Rotationskörper (25) und dem zweiten Rotationskörper (26) größer ist als ein Abstand zwischen dem ersten Rotationskörper und dem zweiten Rotationskörper während der Verarbeitung.

4. Verfahren für die Herstellung einer Verbundlage eines absorbierenden Artikels nach einem der Ansprüche 1 bis 3, wobei,
während die Rotation des ersten Rotationskörpers (25) gestoppt ist,
ein Förderweg, der sich unmittelbar vor dem Kontakt des ersten Rotationskörpers befindet, eines Förderwegs der ersten kontinuierlichen Lage, die auf der Seite des ersten Rotationskörpers der ersten kontinuierlichen Lage (21) und der zweiten kontinuierlichen Lage (22) positioniert ist, in einem Zustand vorliegt, der zu einer Richtung, getrennt von dem ersten Rotationskörper, geändert ist.

5. Verfahren für die Herstellung einer Verbundlage eines absorbierenden Artikels nach einem der Ansprüche 1 bis 4, wobei
eine Fördergeschwindigkeit zur Förderung der ersten kontinuierlichen Lage (21), der zweiten kontinuierlichen Lage (22) und des elastischen Bands (23) nach der Verarbeitung größer ist als vor der Verarbeitung.

6. Verfahren für die Herstellung einer Verbundlage eines absorbierenden Artikels nach einem der Ansprüche 1 bis 5, wobei
das elastische Band (23) zwischen der ersten kontinuierlichen Lage (21) und der zweiten kontinuierlichen Lage (22) in einem Zustand, mit einem Haftmittel angebracht, angeordnet ist.

7. Verfahren für die Herstellung einer Verbundlage eines absorbierenden Artikels nach einem der Ansprüche 1 bis 6, wobei
ein Haftmittel in einem Zustand vorliegt, in dem es zwischen dem elastischen Band (23) und der ersten kontinuierlichen Lage, die auf der Seite des ersten Rotationskörpers der ersten kontinuierlichen Lage (21) und der zweiten kontinuierlichen Lage (22) positioniert ist, angebracht ist,
der erste Rotationskörper ausgesparte Teile (255) zwischen den ersten vorstehenden Teilen (251) und den zweiten vorstehenden Teilen (252) einschließt und
sich die erste kontinuierliche Lage, die auf der Seite des ersten Rotationskörpers der ersten kontinuierlichen Lage (21) und der zweiten kontinuierlichen Lage (22) positioniert ist, getrennt von einem am meisten ausgesparten Abschnitt der ausgesparten Teile (255) durch einen vorgegebenen Abstand befindet.

8. Verfahren für die Herstellung einer Verbundlage eines absorbierenden Artikels nach einem der Ansprüche 1 bis 7, wobei
mindestens ein vorstehender Teil des ersten vorstehenden Teils (251) und des zweiten vorstehenden Teils (252) eine erste schräge Oberfläche (258) und eine zweite schräge Oberfläche (259) in einem Spitzenendabschnitt in einer vorstehenden Richtung einschließt,
in einer Richtung der Rotation des ersten Rotationskörpers (25) die erste schräge Oberfläche (258) zu einer nachgeschalteten Seite als die zweite schräge Oberfläche (259) bereitgestellt ist und,
wenn eine Ebene ("a"), die die erste schräge Oberfläche (258) einschließt, eine erste Ebene ist und
eine Ebene ("b"), die die zweite schräge Oberfläche (259) einschließt, eine zweite Ebene ist,
eine Position, in der sich die erste Ebene und die zweite Ebene kreuzen, in der Richtung der Rotation zu einer vorgeschalteten Seite als einer zentralen Position des mindestens einen vorstehenden Teils positioniert ist.

9. Verfahren für die Herstellung einer Verbundlage eines absorbierenden Artikels nach einem der Ansprüche 1 bis 8, wobei
vor der Verarbeitung die erste kontinuierliche Lage, die mindestens auf der Seite des ersten Rotationskörpers der ersten kontinuierlichen Lage (21) und der zweiten kontinuierlichen Lage (22) positioniert ist, erwärmt wird.

10. Verfahren für die Herstellung einer Verbundlage eines absorbierenden Artikels nach einem der Ansprüche 1 bis 9, wobei
der erste Rotationskörper (25) Folgendes einschließt: eine erste vorstehende Teilgruppe (253), die aus einer Vielzahl von vorstehenden Teilen entlang einer Umfangsrichtung ausgebildet ist, und eine zweite vorstehende Teilgruppe (254), die aus einer Vielzahl von vorstehenden Teilen entlang einer Umfangsrichtung ausgebildet ist,
die erste vorstehende Teilgruppe (253) die ersten vorstehenden Teile (251) einschließt,
die zweite vorstehende Teilgruppe (254) die zweiten vorstehenden Teile (252) einschließt und
jedes vorstehende Teil der ersten vorstehenden Teilgruppe (253) und jedes vorstehende Teil der zweiten vorstehenden Teilgruppe (254) in Positionen angeordnet sind, die sich in einer axialen Richtung und der Umfangsrichtung voneinander unterscheiden.

11. Verfahren für die Herstellung einer Verbundlage eines absorbierenden Artikels nach einem der Ansprüche 1 bis 10, wobei
in einer bestimmten Position in einer Richtung der Rotation des ersten Rotationskörpers (25)
eine Vielzahl der elastischen Bänder (23) zwischen der ersten kontinuierlichen Lage (21) und der zweiten kontinuierlichen Lage (22) angeordnet ist,
die erste kontinuierliche Lage, die zweite kontinuierliche Lage und die elastischen Bänder zu einer anderen Position in der Richtung der Rotation unter Verwendung des ersten Rotationskörpers (25) gefördert werden und
die Verarbeitung durch Zusammenpressen der ersten kontinuierlichen Lage (21) und der zweiten kontinuierlichen Lage (22) in der anderen Position durchgeführt wird.

12. Vorrichtung (2) für die Herstellung einer Verbundlage (20) eines absorbierenden Artikels (1), um eine Verbundlage herzustellen, die Folgendes einschließt: eine erste kontinuierliche Lage (21), eine zweite kontinuierliche Lage (22) und ein elastisches Band (23), das zwischen der ersten kontinuierlichen Lage und der zweiten kontinuierlichen Lage angeordnet ist, wobei die Vorrichtung für die Herstellung Folgendes umfasst:
ein Förderteil, das in einer fortführenden Richtung die erste kontinuierliche Lage (21), die zweite kontinuierliche Lage (22) und das elastische Band (23), die Materialien sind, in einem Zustand fördert, wo das elastische Band, das zwischen der ersten kontinuierlichen Lage und der zweiten kontinuierlichen Lage angeordnet ist, zumindest teilweise zwischen ersten vorstehenden Teilen (251) und zweiten vorstehenden Teilen (252) durch Bewirken einer Rotation eines rotierbaren ersten Rotationskörpers (25) eingefügt ist, wobei der erste Rotationskörper (25) die ersten vorstehenden Teile (251) und die zweiten vorstehenden Teile (252) einschließt, die radial nach außen vorstehen;
ein Verarbeitungsteil, das die erste kontinuierliche Lage (21) und die zweite kontinuierliche Lage (22) durch Zusammenpressen der ersten kontinuierlichen Lage und der zweiten kontinuierlichen Lage zwischen einem rotierbaren zweiten Rotationskörper (26) und den ersten vorstehenden Teilen (251) und den zweiten vorstehenden Teilen (252) während des Förderns mit dem ersten Rotationskörper (25) verarbeitet; und
ein Hebeteil (24), das die erste kontinuierliche Lage (21) und das elastische Band (23) zu einer oberen Seite in einer Dickenrichtung anhebt, um diese getrennt von dem ersten Rotationskörper zu positionieren, wenn die Rotation des ersten Rotationskörpers gestoppt wird;
wobei die Dickenrichtung der Materialien, die fortführende Richtung und eine Breitenrichtung in einer orthogonalen Beziehung zueinander stehen.

## Revendications

1. Procédé de fabrication d'une feuille composite (20) d'un article absorbant (1) pour fabriquer une feuille composite comprenant une première feuille continue (21), une deuxième feuille continue (22), et un fil élastique (23) destiné à être agencé entre la première feuille continue et la deuxième feuille continue, le procédé de fabrication d'une feuille composite d'un article absorbant comportant les étapes consistant à :
transporter, dans une direction allant dans le sens de la continuité, la première feuille continue (21), la deuxième feuille continue (22), et le fil élastique (23), qui sont des matières, dans un état dans lequel le fil élastique agencé entre la première feuille continue et la deuxième feuille continue est intercalé au moins partiellement entre des premières parties faisant saillie (251) et des deuxièmes parties faisant saillie (252), en amenant un premier corps de rotation rotatif (25) à tourner, le premier corps de rotation (25) comprenant les premières parties faisant saillie (251) et les deuxièmes parties faisant saillie (252) qui font saillie vers l'extérieur dans le sens radial ; et
effectuer un traitement au niveau de la première feuille continue (21) et de la deuxième feuille continue (22) en prenant en sandwich la première feuille continue et la deuxième feuille continue entre un deuxième corps de rotation rotatif (26) et les premières parties faisant saillie (251) et les deuxièmes parties faisant saillie (252), au cours du transport au moyen du premier corps de rotation (25) ;
dans lequel
une direction allant dans le sens de l'épaisseur des matières, la direction allant dans le sens de la continuité, et une direction allant dans le sens de la largeur se trouvent dans une relation orthogonale les unes par rapport aux autres ; et
alors que la rotation du premier corps de rotation (25) est arrêtée, la première feuille continue, positionnée sur le côté premier corps de rotation de la première feuille continue (21) et de la deuxième feuille continue (22), et le fil élastique (23) sont placés à distance du premier corps de rotation et soulevés vers le haut jusque sur un côté supérieur dans la direction allant dans le sens de l'épaisseur au moyen d'une partie de levage (24).

2. Procédé de fabrication d'une feuille composite d'un article absorbant selon la revendication 1, dans lequel
au cours du traitement, les premières parties faisant saillie (251) et les deuxièmes parties faisant saillie (252) chauffent la première feuille continue (21) et la deuxième feuille continue (22).

3. Procédé de fabrication d'une feuille composite d'un article absorbant selon la revendication 1 ou la revendication 2, dans lequel
alors que la rotation du premier corps de rotation (25) est arrêtée, une distance entre le premier corps de rotation (25) et le deuxième corps de rotation (26) est plus large qu'une distance entre le premier corps de rotation et le deuxième corps de rotation au cours du traitement.

4. Procédé de fabrication d'une feuille composite d'un article absorbant selon l'une quelconque des revendications 1 à 3, dans lequel
alors que la rotation du premier corps de rotation (25) est arrêtée,
un chemin de transfert situé directement avant la mise en contact avec le premier corps de rotation, d'un chemin de transfert de la première feuille continue positionnée sur le côté premier corps de rotation de la première feuille continue (21) et de la deuxième feuille continue (22), est dans un état changé dans une direction à distance du premier corps de rotation.

5. Procédé de fabrication d'une feuille composite d'un article absorbant selon l'une quelconque des revendications 1 à 4, dans lequel
une vitesse de transport pour transporter la première feuille continue (21), la deuxième feuille continue (22), et le fil élastique (23) est plus rapide après le traitement qu'avant le traitement.

6. Procédé de fabrication d'une feuille composite d'un article absorbant selon l'une quelconque des revendications 1 à 5, dans lequel
le fil élastique (23) est agencé entre la première feuille continue (21) et la deuxième feuille continue (22) dans un état attaché au moyen d'un adhésif.

7. Procédé de fabrication d'une feuille composite d'un article absorbant selon l'une quelconque des revendications 1 à 6, dans lequel
un adhésif est dans un état attaché entre le fil élastique (23) et la première feuille continue positionnée sur le côté premier corps de rotation de la première feuille continue (21) et de la deuxième feuille continue (22),
le premier corps de rotation comprend des parties en retrait (255) entre les premières parties faisant saillie (251) et les deuxièmes parties faisant saillie (252), et
la première feuille continue, positionnée sur le côté premier corps de rotation de la première feuille continue (21) et de la deuxième feuille continue (22), est située à distance d'une partie la plus en retrait des parties en retrait (255) selon une distance prédéterminée.

8. Procédé de fabrication d'une feuille composite d'un article absorbant selon l'une quelconque des revendications 1 à 7, dans lequel
au moins une partie faisant saillie de la première partie faisant saillie (251) et de la deuxième partie faisant saillie (252) comprend une première surface inclinée (258) et une deuxième surface inclinée (259) dans une partie d'extrémité de bout dans une direction allant dans le sens faisant saillie,
dans une direction allant dans le sens de la rotation du premier corps de rotation (25), la première surface inclinée (258) est mise en œuvre au niveau d'un côté en aval par rapport à la deuxième surface inclinée (259), et
quand un plan (« a ») comprenant la première surface inclinée (258) est un premier plan, et
quand un plan (« b ») comprenant la deuxième surface inclinée (259) est un deuxième plan,
une position dans laquelle le premier plan et le deuxième plan se croisent, dans la direction allant dans le sens de la rotation, est positionnée au niveau d'un côté en amont par rapport à une position centrale de ladite au moins une partie faisant saillie.

9. Procédé de fabrication d'une feuille composite d'un article absorbant selon l'une quelconque des revendications 1 à 8, dans lequel
avant le traitement, la première feuille continue, positionnée au moins sur le côté premier corps de rotation de la première feuille continue (21) et de la deuxième feuille continue (22) est chauffée.

10. Procédé de fabrication d'une feuille composite d'un article absorbant selon l'une quelconque des revendications 1 à 9, dans lequel
le premier corps de rotation (25) comprend un premier groupe de parties faisant saillie (253) formé à partir d'une pluralité de parties faisant saillie le long d'une direction circonférentielle et un deuxième groupe de parties faisant saillie (254) formé à partir d'une pluralité de parties faisant saillie le long d'une direction circonférentielle,
le premier groupe de parties faisant saillie (253) comprend les premières parties faisant saillie (251),
le deuxième groupe de parties faisant saillie (254) comprend les deuxièmes parties faisant saillie (252), et
chaque partie faisant saillie du premier groupe de parties faisant saillie (253) et chaque partie faisant saillie du deuxième groupe de parties faisant saillie (254) sont agencées dans des positions différentes l'une de l'autre dans une direction axiale et la direction circonférentielle.

11. Procédé de fabrication d'une feuille composite d'un article absorbant selon l'une quelconque des revendications 1 à 10, dans lequel
dans une certaine position dans une direction allant dans le sens de la rotation du premier corps de rotation (25),
une pluralité de fils élastiques (23) sont agencés entre la première feuille continue (21) et la deuxième feuille continue (22),
la première feuille continue, la deuxième feuille continue, et les fils élastiques sont transportés vers une autre position dans la direction allant dans le sens de la rotation au moyen du premier corps de rotation (25), et
le traitement est effectué en prenant en sandwich la première feuille continue (21) et la deuxième feuille continue (22) dans l'autre position.

12. Appareil (2) de fabrication d'une feuille composite (20) d'un article absorbant (1) pour fabriquer une feuille composite comprenant une première feuille continue (21), une deuxième feuille continue (22), et un fil élastique (23) agencé entre la première feuille continue et la deuxième feuille continue, l'appareil de fabrication comportant :
une partie de transport qui transporte, dans une direction allant dans le sens de la continuité, la première feuille continue (21), la deuxième feuille continue (22), et le fil élastique (23), qui sont des matières, dans un état dans lequel le fil élastique agencé entre la première feuille continue et la deuxième feuille continue est intercalé au moins partiellement entre des premières parties faisant saillie (251) et des deuxièmes parties faisant saillie (252), en amenant un premier corps de rotation rotatif (25) à tourner, le premier corps de rotation (25) comprenant les premières parties faisant saillie (251) et les deuxièmes parties faisant saillie (252) qui font saillie vers l'extérieur dans le sens radial ; et
une partie de traitement qui traite la première feuille continue (21) et la deuxième feuille continue (22) en prenant en sandwich la première feuille continue et la deuxième feuille continue entre un deuxième corps de rotation rotatif (26) et les premières parties faisant saillie (251) et les deuxièmes parties faisant saillie (252), au cours du transport au moyen du premier corps de rotation (25) ; et
une partie de levage (24) qui soulève vers le haut la première feuille continue (21) et le fil élastique (23) jusque sur un côté supérieur dans une direction allant dans le sens de l'épaisseur pour les placer à distance du premier corps de rotation quand la rotation du premier corps de rotation est arrêtée ;
dans lequel la direction allant dans le sens de l'épaisseur des matières, la direction allant dans le sens de la continuité, et une direction allant dans le sens de la largeur se trouvent dans une relation orthogonale les unes par rapport aux autres.
